# EUROPEAN PATENT APPLICATION

(11) **EP 3 091 025 A1**
(43) Date of publication of application: **09.11.2016**
(21) Application number: 15167037.9
(22) Date of filing: 08.05.2015
(51) Int. Cl.: C07H 15/203, B67D 1/07

(54) **BEVERAGE DISPENSING EQUIPMENT**

(71) Applicant: The Provost, Fellows, Foundation Scholars, and The Other Members of Board, of The College of The Holy and Undivided Trinity of Queen Elizabeth, Dublin 2 (IE)
(72) Inventor: Colavita, Paula E., Dublin 2 (IE); Scanalan, Eoin M., Dublin 2 (IE); Angione, Maria Daniela, Co. Dublin (IE); Duff, Thomas, Dublin 9 (IE)
(74) Representative: Tomkins & Co

(57) **Abstract**

Apparatus and methods for reducing biofouling in drink such as beer dispensing equipment are described.

## Description

### Field of the Invention

The present invention relates to drinking liquid, for example, beverage dispensing equipment.

### Background to the Invention

Drinking liquids including beverages which are non-alcoholic such as soft drinks (for example carbonated drinks) and hard or alcohol-containing drinks are often conveyed from a reservoir through dispensing lines to a dispenser for example a dispensing tap. Such an arrangement is often present in premises with a bar.

For example beer and related beverages such a draft (also spelled draught) beverages are often conveyed through dispensing lines such as ducts or pipes from a reservoir such as a keg to an outlet such as a tap for dispensing purposes. Contamination of drink, for example beverage, such as beer, dispensing equipment, including pipes, ducting, valves, taps and restrictor disks through biofouling is presently combatted in industry by regular cleaning. This process generally involves disassembly of dispensing equipment such as pipelines and taps and flushing a cleaning fluid therethrough. This process can be tedious and costly, in terms of loss of product for example beer, down time, and the expense of cleaning products.

Pipelines conveying both alcoholic and non-alcoholic beverages are subject to biofouling. In addition to, for example, beer dispensing lines, pipelines conveying cider, soft drinks, juices, water and dairy products such as milk can be similarly affected.

Several approaches have been developed to combat biofouling in beer dispensing equipment.

US5645697 describes a method for preventing contaminant build-up in beer supply line by locating electromagnetic field generating means adjacent a section of a beer dispensing line, applying a frequency modulated square wave voltage signal to said electromagnetic field generating means to generate a varying frequency electromagnetic field in that section of the dispensing line. The authors present several explanations for the reduction in contaminants including that the applied electromagnetic field created in the pipe or duct carrying the beer interferes with electron transport within bacterial cells, leading to cell stasis or death; and/or that the electromagnetic field may block uptake of key nutrients thereby inhibiting bacterial cell growth.

GB236106 is also concerned with the application of an electromagnetic field about beer dispensing equipment.

WO2008035051 describes a fluid conveying conduit comprising a coil for producing a magnetic field within the body of the conduit. The authors describe disadvantages associated with wrapping coils for electromagnetic generation about beer lines, for example that said coils are prone to damage, particularly when lines are being moved. The system of WO2008035051 has the magnetic coil in-built in the conduit thereby reducing the likelihood of damage to the coil.

Biofouling in beverage dispensing equipment which carry milk containing products is particularly problematic, for example, milk spoiling in coffee machines will compromise the quality, flavour and taste of coffee produced thereby. Saint-Gobain® have developed a platinum-cured silicon tubing under the trade name Tygon® SPT-50 LF to address this issue.

Notwithstanding the various methods and apparatus that are available to combat biofouling in beverage dispensing equipment, it is desirable to provide an alternative construction of beverage dispensing equipment, and an alternative approach for combatting biofouling.

### Summary of the Invention

In one aspect, the present invention provides beverage dispensing equipment having a surface; said surface having a disaccharide immobilised thereon; wherein the disaccharide has a linker moiety covalently bound thereto; the linker moiety is disposed between the surface and the disaccharide, and the linker moiety comprises a carbon atom that forms a covalent bond with an atom on the surface.

Also provided for is the use of an article, in beverage dispensing equipment; said article having a surface; said surface having a disaccharide immobilised thereon; wherein the disaccharide has a linker moiety covalently bound thereto; the linker moiety is disposed between the surface and the disaccharide, and the linker moiety comprises a carbon atom that forms a covalent bond with an atom on the surface.

In particular the present invention provides for use of an article, in beverage dispensing equipment such as beverage dispensing lines, beverage dispensing taps and restrictor disks used in beverage dispensing taps.

Advantageously, surfaces having a disaccharide immobilised thereon as described above are resistant to biofouling. A reduction of biofouling in beverage dispensing equipment, for example in beer dispensing equipment, is particularly desirable. To combat biofouling, traditional beer dispensing equipment needs to be cleaned regularly. Advantageously the surfaces of the present invention, significantly decrease biofouling in beer dispensing equipment, thereby reducing the frequency of which said equipment requires cleaning, thus potentially leading to reduced costs and improved efficiencies.

As used herein the term "beverage" shall be construed as relating to alcoholic and non-alcoholic drinks including but not limited to beer, cider, soft drinks, drinks comprising dairy products, coffee, tea, juices, and water. The beverage may be carbonated or non-carbonated.

As used herein the term "beer" shall be construed as relating to at least to beers prepared from mashes prepared from malted cereals as well as mashes prepared from unmalted cereals, and mashes prepared from a mixture of malted and unmalted cereals. The term "beer" also refers to beers prepared with adjuncts and beers with all possible alcohol contents; the term "beer" for example can be construed as relating to beer, lager, ale, porter, stout, and like such liquids.

The term "disaccharide" shall be construed as relating to natural and synthetic carbohydrates that are composed of two monosaccharide residues which are joined by a glycosidic bond, including all stereoisomeric and enantiomeric forms thereof. Representative examples of disaccharides include maltose, sucrose, lactose, lactulose, trehalose, cellobiose, isomaltose, gentiobiose, laminarabiose, chitobiose, xylobiose, inulobiose, mannobiose, hyalobiouronic acid, chondrosine, and cellobiouronic acid.

It will be understood that the term disaccharides are composed of monosaccharides which include molecules that have a 5 and 6 membered saturated ring having the substituents on the ring such as the groups as defined herein and each ring contains one heteroatom, which forms part of the ring structure. Suitably, the heteroatom may be O, S or N. Accordingly, disaccharides comprising thiosugars and iminosugars also fall within the scope of the invention. A natural disaccharide shall be considered as an organic compound (synthesised *de novo* or isolated from a natural source), which is capable of being biosynthesised by living organisms, and which has the empirical formula Cₘ(H₂O)ₙ where m and n can be the same or different.

Disaccharides suitable for use in the present invention can be represented by the general formula:

M₁-X-M₂

wherein M₁ and M₂ are each independently C₄ or C₅ monosaccharide sugars, which can be cyclic or acyclic, and are optionally substituted with OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy;
wherein at least one of M₁ or M₂ is a heterocyclic sugar ring, comprising O, S or NR in the ring; and wherein X is O, S or NR;
wherein each R is independently selected from the group consisting of H, a C₁-C₁₀ aliphatic chain and wherein X is bonded in the anomeric position of either M₁ or M₂.

For example, disaccharides of the present invention may have the following formula: 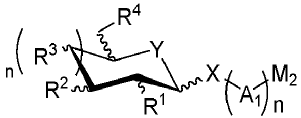 wherein M₂ is a C₅ or C₆ monosaccharide sugar, which can be cyclic or acyclic, and is optionally substituted with OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy;
wherein Y is O, S or NR;
X is O, S or NR;
each n can independently be 0 or 1;
A₁ is -CH₂-;
wherein each of R¹, R², R³, and R⁴ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy,
and wherein each R is independently selected from the group consisting of H, a C₁-C₁₀ aliphatic chain.

Preferably, Y is O and X is O.

Other suitable disaccharides for use in the present invention can be represented by the formula: 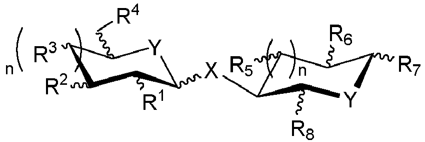 wherein each of R¹, R², R³, R⁴, R⁵, R⁶, R⁷, and R⁸ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy,
wherein each Y is independently selected from the group consisting of O, S or NR;
X is O, S or NR;
each n may independently be 0 or 1;
and wherein each R is independently selected from the group consisting of H, a C₁-C₁₀ aliphatic chain.

Suitably each Y and X are O.

For example, compounds having the following formula may be used:

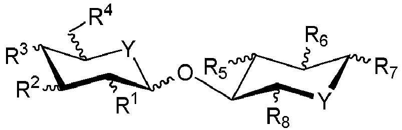

Preferably, Y is O.

Such as:

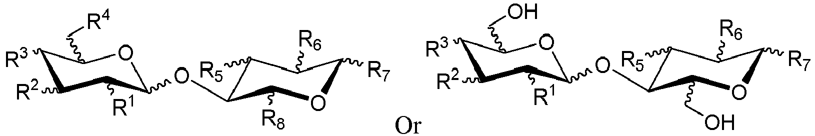

Specifically:

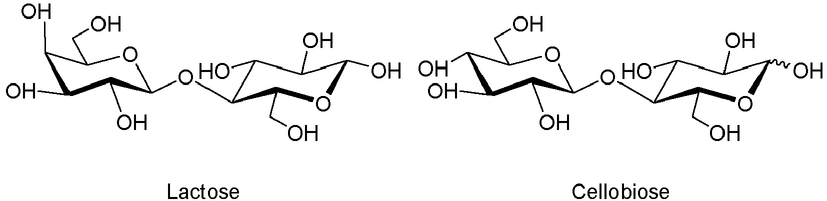

Disaccharides having any combination of R1, R2, R3, R4, R5, R6, R7 and R8 substituents are suitable and encompassed by the present invention.

A further representative formula for disaccharides suitable for use in the present invention is shown below:

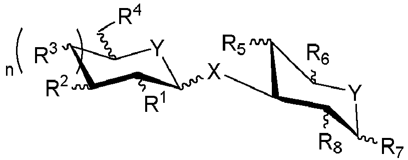

Y is O is preferred. Similarly Y is O and X is O is preferred.

Specifically:

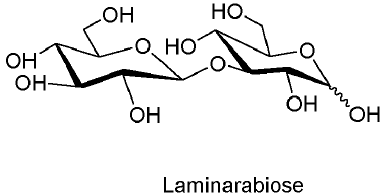

Another representative formula for disaccharides suitable for use in the present invention is shown below: 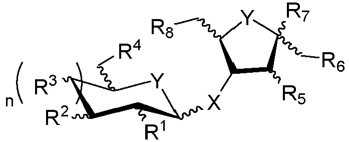 wherein each of R¹, R², R³, R⁴, R⁵, R⁶, R⁷, and R⁸ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy,
wherein each Y is independently selected from the group consisting of O, S or NR;
X is O, S or NR;
n may independently be 0 or 1;
and wherein each R is independently selected from the group consisting of H, a C₁-C₁₀ aliphatic chain.

Y is O is preferred. Similarly Y is O and X is O is preferred.

Specific examples include:

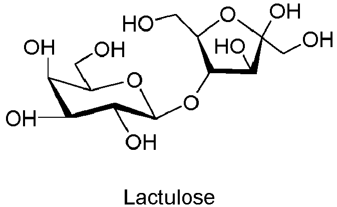

Disaccharides according to the above-mentioned formulae, having any combination of R¹, R², R³, R⁴, R⁵, R⁶, R⁷ and R⁸ substituents are suitable for use in, and encompassed by, the present invention.

In one embodiment the disaccharide and linker immobilised on the surface of the beverage dispensing equipment may have the following general formula: 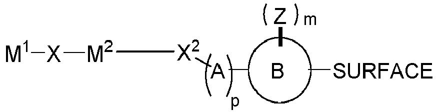 wherein M¹ and M² are each independently C₄ or C₅ monosaccharide sugars, which can be cyclic or acyclic, and are optionally substituted with OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy;
wherein at least one of M₁ or M₂ is a heterocyclic sugar ring, comprising O, S or NR in the ring; and wherein X is O, S or NR;
wherein each R is independently selected from the group consisting of H, a C₁-C₁₀ aliphatic chain and wherein X is bonded in the anomeric position of either M¹ or M²; wherein X² is O, S, NH or CH₂;
m can be 0 to 4;
each Z can be independently selected from hydroxy, Cl, Br, I, F, cyano, C₁-C₅ alkoxy, and C₁-C₅ thioalkoxy;
B can be C₅-C₂₀ aryl, or C₃-C₂₀ heteroaryl;
A can be C₁-C₂₀ aliphatic, or C₁-C₂₀ heteroaliphatic; and p can be 0 or 1;
and whereby B comprises a carbon atom that forms a covalent bond with an atom on the surface.

For example, in one embodiment the disaccharide and linker immobilised on the surface may have the following formula: 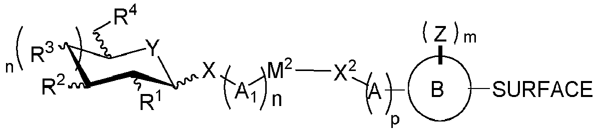 wherein M₂ is a C₅ or C₆ monosaccharide sugar, which can be cyclic or acyclic, and is optionally substituted with OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy;
wherein Y is O, S or NR; X is O, S or NR;
each n can independently be 0 or 1;
A₁ is -CH₂-;
wherein each of R¹, R², R³, and R⁴ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy;
wherein each R is independently selected from the group consisting of H, a C₁-C₁₀ aliphatic chain;
X² is O, S, NH or CH₂;
m can be 0 to 4;
each Z can be independently selected from hydroxy, Cl, Br, I, F, cyano, C₁-C₅ alkoxy, and C₁-C₅ thioalkoxy;
B can be C₅-C₂₀ aryl, or C₃-C₂₀ heteroaryl;
A can be C₁-C₂₀ aliphatic, or C₁-C₂₀ heteroaliphatic; and
p can be 0 or 1;
and whereby B comprises a carbon atom that forms a covalent bond with an atom on the surface.

In another embodiment, the disaccharide and linker immobilised on the surface may have the following formula: 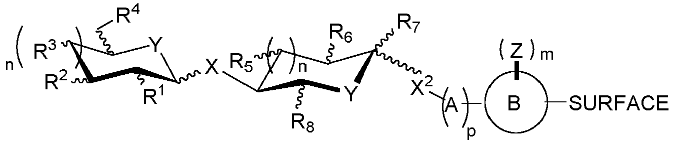 wherein each of R¹, R², R³, R⁴, R⁵, R⁶, R⁷, and R⁸ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy,
wherein each Y is independently selected from the group consisting of O, S or NR; X is O, S or NR;
each n may independently be 0 or 1;
and wherein each R is independently selected from the group consisting of H, a C₁-C₁₀ aliphatic chain; and
X² is O, S, NH or CH₂;
m can be 0 to 4;
each Z can be independently selected from hydroxy, Cl, Br, I, F, cyano, C₁-C₅ alkoxy, and C₁-C₅ thioalkoxy;
B can be C₅-C₂₀ aryl, or C₃-C₂₀ heteroaryl;
A can be C₁-C₂₀ aliphatic, or C₁-C₂₀ heteroaliphatic; and
p can be 0 or 1;
and whereby B comprises a carbon atom that forms a covalent bond with an atom on the surface.

For example, the disaccharide and linker immobilised on the surface may have the following formula: 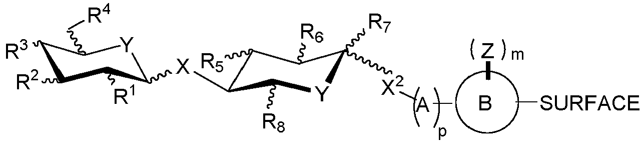 wherein each of R¹, R², R³, R⁴, R⁵, R⁶, R⁷, and R⁸ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bis*alkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy,
wherein each Y is independently selected from the group consisting of O, S or NR;
X is O, S or NR;
each n may independently be 0 or 1;
and wherein each R is independently selected from the group consisting of H, a C₁-C₁₀ aliphatic chain; and
X² is O, S, NH or CH₂;
m can be 0 to 4;
each Z can be independently selected from hydroxy, Cl, Br, I, F, cyano, C₁-C₅ alkoxy, and C₁-C₅ thioalkoxy;
B can be C₅-C₂₀ aryl, or C₃-C₂₀ heteroaryl;
A can be C₁-C₂₀ aliphatic, or C₁-C₂₀ heteroaliphatic; and
p can be 0 or 1;
and whereby B comprises a carbon atom that forms a covalent bond with an atom on the surface.

Suitably, R¹, R², R³, R⁴, R⁵, R⁶, and R⁸ may be OH, X and Y may be O.

In one embodiment R¹, R², R³, R⁴, R⁵, R⁶, and R⁸ are OH, and R⁷ is H; X and Y are O and X² is O.

Suitably, when Y is N or S, it is preferred that at least two of R¹, R², R³, and R⁴⁻ are -OH, and that at least two of R⁵, R⁶, R⁷ and R⁸ are -OH. More preferably still, at least at least three of R¹, R², R³, and R⁴ are -OH. More preferably still, all four of R¹, R², R³, and R⁴ are -OH. In some embodiment, it is preferred that R4 is -OH.

B can be can be selected from the group consisting of phenyl, pyridyl, thienyl, pyrollyl, pyrazyl, pyrimidyl, imidazolyl, indolyl, quinolyl, and isoquinolyl. For example, B may be phenyl.

In one embodiment the disaccharide and linker immobilised on the surface have the following formula:

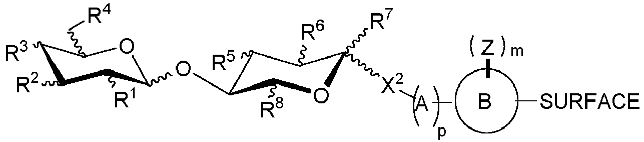

Preferably, R⁷ is H, R⁸ is -CH₂OH and at least 4 of R¹, R², R³, R⁴, R⁵, and R⁶ are OH. More preferably X² is additionally O.

In one embodiment, R⁷ is H, R⁸ is -CH₂OH and at least 4 of R¹, R², R³, R⁴, R⁵, and R⁶ are OH, X² is O, p is 0, and B is phenyl.

For example, the disaccharide and linker immobilised on the surface may be selected from the group consisting of:

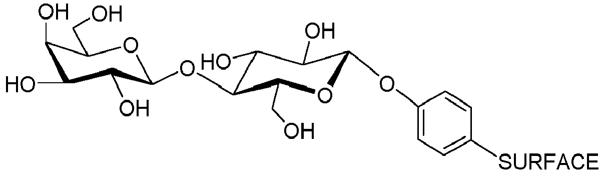

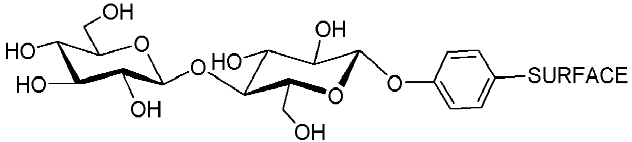

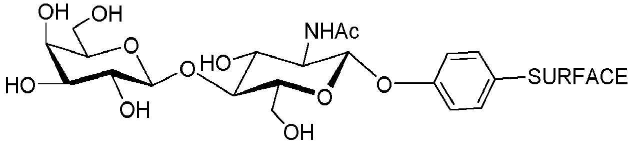

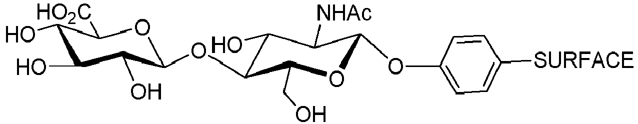

In another embodiment, the disaccharide and linker immobilised on the surface may have the following formula: 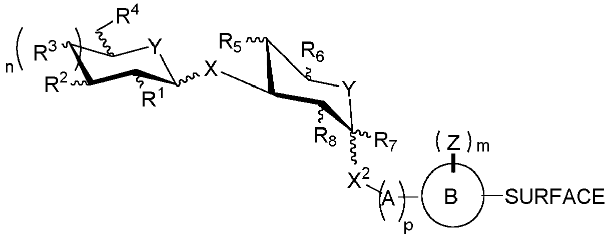 wherein each of R¹, R², R³, R⁴, R⁵, R⁶, R⁷, and R⁸ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy,
wherein each Y is independently selected from the group consisting of O, S or NR; X is O, S or NR;
n may independently be 0 or 1;
and wherein each R is independently selected from the group consisting of H, a C₁-C₁₀ aliphatic chain; and
X² is O, S, NH or CH₂;
m can be 0 to 4;
each Z can be independently selected from hydroxy, Cl, Br, I, F, cyano, C₁-C₅ alkoxy, and C₁-C₅ thioalkoxy;
B can be C₅-C₂₀ aryl, or C₃-C₂₀ heteroaryl;
A can be C₁-C₂₀ aliphatic, or C₁-C₂₀ heteroaliphatic; and
p can be 0 or 1;
and whereby B comprises a carbon atom that forms a covalent bond with an atom on the surface.

Suitably, n is 1, R¹, R², R³, R⁴, R⁵, and R⁸ may be OH, and X and Y may be O.

In one embodiment R¹, R², R³, R⁴, R⁵, and R⁸ are OH, and R⁷ is H; R⁸ is-CH₂OH; X and Y are O and X² is O.

Suitably, when Y is N or S, it is preferred that at least two of R¹, R², R³, and R⁴⁻ are -OH, and that at least two of R⁵, R⁶, R⁷ and R⁸ are -OH. More preferably still, at least at least three of R¹, R², R³, and R⁴ are -OH. More preferably still, all four of R¹, R², R³, and R⁴ are -OH. In some embodiment, it is preferred that R4 is -OH.

B can be can be selected from the group consisting of phenyl, pyridyl, thienyl, pyrollyl, pyrazyl, pyrimidyl, imidazolyl, indolyl, quinolyl, and isoquinolyl. For example, B may be phenyl.

In one embodiment, the disaccharide and linker immobilised on the surface has the formula:

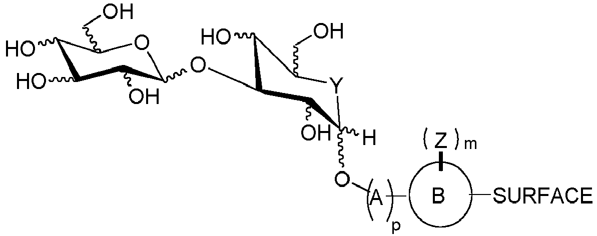

In one embodiment the disaccharide and linker immobilised on the surface has the formula:

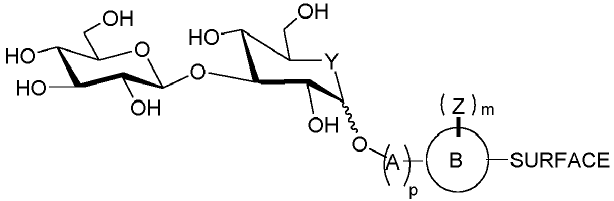

For example, in one embodiment the disaccharide and linker immobilised on the surface has the formula:

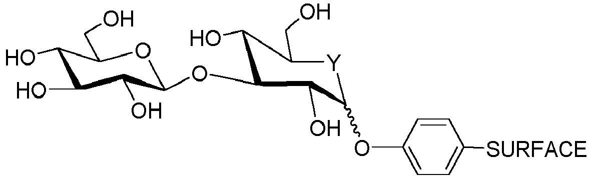

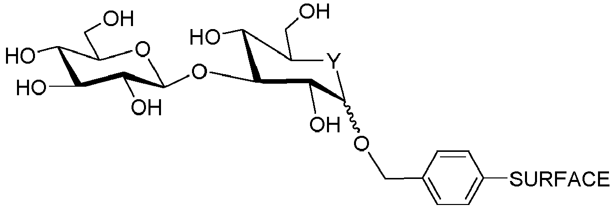

In yet a further embodiment the disaccharide and linker immobilised on the surface has the formula: 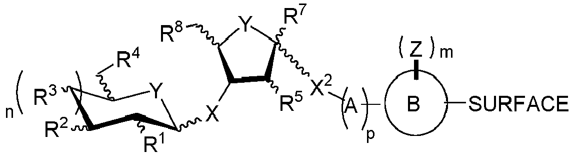 or 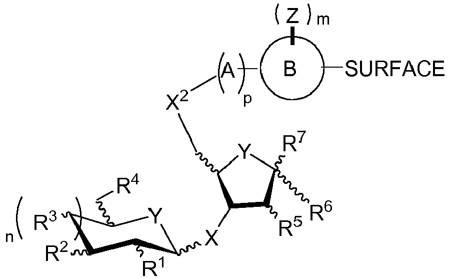 wherein each of R¹, R², R³, R⁴, R⁵, R⁶, R⁷, and R⁸ can be the same or different and may be selected from the group consisting of OH, H, NH₂, F, SH, C₂-C₃₀ carbon esters, C₂-C₃₀ amides, C₂-C₃₀ phosphate esters, C₁-C₁₀ amino alkyl, C₂-C₂₀ *bi*salkyl amino, C₁-C₁₀ alkoxy, C₁-C₁₀ thioalkoxy, C₅-C₂₀ aryloxy, C₅-C₂₀ thioaryloxy, C₅-C₂₀ heteroaryloxy, C₅-C₂₀ thioheteroaryloxy,
wherein each Y is independently selected from the group consisting of O, S or NR;
X is O, S or NR;
n may independently be 0 or 1;
and wherein each R is independently selected from the group consisting of H, a C₁-C₁₀ aliphatic chain;
X² is O, S, NH or CH₂;
m can be 0 to 4;
each Z can be independently selected from hydroxy, Cl, Br, I, F, cyano, C₁-C₅ alkoxy, and C₁-C₅ thioalkoxy;
B can be C₅-C₂₀ aryl, or C₃-C₂₀ heteroaryl;
A can be C₁-C₂₀ aliphatic, or C₁-C₂₀ heteroaliphatic; and
p can be 0 or 1;

and whereby B comprises a carbon atom that forms a covalent bond with an atom on the surface. The surface of the present invention will preferably be a reducing surface relative to the diazonium cation, *i.e.* the surface will provide electrons to reduce the diazonium cation. For example, electron donation from a surface state to the molecular state, *i.e.* the diazonium cation, should be thermodynamically favoured.

For surfaces where the standard reduction potential is measurable, the diazonium cation utilised in the method of the present invention should have a standard reduction potential greater than the particular surface to which the carbohydrate is to be bound. References to standard reduction potentials in this specification indicate the tendency of a species to acquire electrons and thereby be reduced. Standard reduction potentials are measured under standard conditions: 25 °C, 1 M concentration, a pressure of 1.01325 × 10⁵ Pa (1 atm) and elements in their pure state.

Alternatively, the reduction of the diazonium cation can be driven electrochemically. Where the reduction of the diazonium cation is driven electrochemically the surface should be conducting. In this case the surface will be selected from the group consisting of metals and semiconductors.

Suitable surfaces for use in the method of the present invention may be selected from the group consisting of metals including Ti, Fe, Cr, Cu, Zn, Al, and alloys thereof containing at least one of said metals, such as stainless steels, brasses, bronzes; tin oxide, SiO₂, titanium oxide, iron oxides, zinc oxides, polymers or plastics such as, polystyrene, polythene, nylon, polytetrafluoroethylene (PTFE), polyestersulfone, polyethylene terephthalate (PET), polyethersulfone (PES), polyvinyl chlorides (PVC), polystyrenes (PS), polyesters, polyepoxides, polyacetates (e.g. polyvinylacetate), polyethylene oxide, polymethylene oxide, polyphenyl oxide, silicones, polybutadiene, polyacrilonitrile, polypropylene (PP), polyethylene (PE), polyvinylidenefluoride (PVDF), polybutylene (PB), perfluoroalkoxy alkanes (PFA), fluorinated ethylene propylene (FEP), ethylene trifluoroethylene (ETFE), polycarbonates (PC), polyestersulfone (PES), polysulfones, Polyetherimide (PEI), polyamides (e.g. Nylon, Aramids), polyimides (e.g. Vespel), poly(vinyl alcohol) (PVA), polyacrylics (e.g. PMMA, PAA), polyoxymethylenes (POM), polyurethanes, polyethylene terephthalate (PET), polytrimethylene terephthalate (PTT), polybutyrate, melamine and combinations thereof.

Particularly preferred metal and alloys are Cu, Fe, Ti, Zn and alloys thereof containing at least one of said metals, such as stainless steel; SiO₂, titanium oxide, iron oxides, zinc oxides.

Suitable surfaces include those substantially made from polystyrene, polythene, polyamides (e.g. nylon), polytetrafluoroethylene (PTFE), polyestersulfone, polyethylene terephthalate (PET), and combinations thereof.

Preferred surfaces for immobilisation of a disaccharide thereto include those made substantially from, a stainless steel material, a polymethylmethacrylate material, a polyamide material, a polyethylene material, a polyacetal material such as polyoxymethylene, or copolymers thereof, an acrylonitrile material and/or a polyvinyl material such as polyvinyl alcohol or polyvinyl chloride.

Particularly preferred surfaces include those made substantially of a polyamide material, such as nylon, for example nylon-4,6, nylon-6,6, nylon-6, nylon-11, nylon-12.

It will be appreciated that the nature of the atom on the surface to which the carbon of the linker moiety is covalently bound, depends on the surface itself. For example, in carbon based surfaces this atom is C, whereby a C-C covalent bond is formed. For metal or metal oxide surfaces this atom is typically an O, whereby a C-O covalent bond is formed.

In a preferred embodiment, the surface comprises carbon such that the linker moiety is bonded to the surface by means of a C-C bond. Advantageously, this results in particularly strong bonding of the carbohydrate to the surface by means of a C-C bond, which is resistant to hydrolysis.

Suitably the surface is made substantially from a polyamide material, a vinyl material, a thermoplastic vinyl material, a polyethylene material, a stainless steel material, or a polyoxymethylene material.

The linker moiety may be covalently bound to the carbohydrate by means of a glycosidic bond. The glycosidic bond may be selected from the group consisting of an *O*-glycosidic bond, an *S*-glycosidic bond, an *N*-glycosidic bond and a C-glycosidic bond. The glycosidic bond may be an anomeric glycosidic bond.

The linker moiety may be selected from the group consisting of C₅-C₂₀ aryl, C₃-C₂₀ heteroaryl, C₅-C₂₀ aryloxy, C₃-C₂₀ heteroaryloxy, C₅-C₂₀ aryl substituted with C₁-C₂₀ aliphatic, C₅-C₂₀ aryl substituted with C₃-C₂₀ cycloaliphatic, C₃-C₂₀ heteroaryl substituted with C₁-C₂₀ aliphatic, C₃-C₂₀ heteroaryl substituted with C₃-C₂₀ cycloaliphatic, C₃-C₂₀ heteroaryl substituted with C₁-C₂₀ heteroaliphatic, and C₃-C₂₀ heteroaryl substituted with C₃-C₂₀ cycloheteroaliphatic, wherein each of the above moieties can be optionally substituted one or more times with at least one of hydroxy, Cl, Br, I, F, cyano, C₁-C₅ alkoxy, and C₁-C₅ thioalkoxy.

As used herein, the term Cₓ-C_{y} aliphatic refers to linear, branched, saturated and unsaturated hydrocarbon chains comprising Cₓ-C_{y} carbon atoms (and includes Cₓ-C_{y} alkyl, Cₓ-C_{y} alkenyl and Cₓ-C_{y} alkynyl). Cₓ-C_{y} heteroaliphatic refers to linear, branched, saturated and unsaturated hydrocarbon chains comprising Cₓ-C_{y} carbon atoms, wherein the carbon atoms are interspaced with heteroatoms such as O, N, and S in no regular order or sequence. Cₓ-C_{y} heteroaliphatic shall be construed as covering polyethers and polythioethers.

Similarly, references to Cₓ-C_{y} alkyl, Cₓ-C_{y} alkenyl and Cₓ-C_{y} alkynyl include linear and branched Cₓ-C_{y} alkyl, Cₓ-C_{y} alkenyl and C_{X}-C_{y} alkynyl.

As used herein, the term "Cₓ-C_{y} cycloaliphatic" refers to unfused, fused, spirocyclic, polycyclic, saturated and unsaturated hydrocarbon rings comprising Cₓ-C_{y} carbon atoms (and includes Cₓ-C_{y} cycloalkyl, Cₓ-C_{y} cycloalkenyl and Cₓ-C_{y} cycloalkynyl).

As used herein, the term aryl/aromatic refers to an aromatic carbocyclic structure which is monocyclic or polycyclic and is unfused or fused. As used herein, the term heterocycle refers to cyclic compounds having as ring members, atoms of at least two different elements. The cyclic compounds may be monocyclic or polycyclic, and unfused or fused.

As used herein, the term heteroaromatic/heteroaryl refers to an aromatic heterocyclic structure having as ring members, atoms of at least two different elements. The heterocycle may be monocyclic or polycyclic, and unfused or fused.

The compounds of the present invention may be found or isolated in the form of esters, salts, hydrates or solvates - all of which are embraced by the present invention.

Where suitable, it will be appreciated that all optional and/or preferred features of one embodiment of the invention may be combined with optional and/or preferred features of another/other embodiment(s) of the invention.

### Brief Description of the Drawings

Embodiments of the invention will be described, by way of example only, with reference to the accompanying drawings in which:

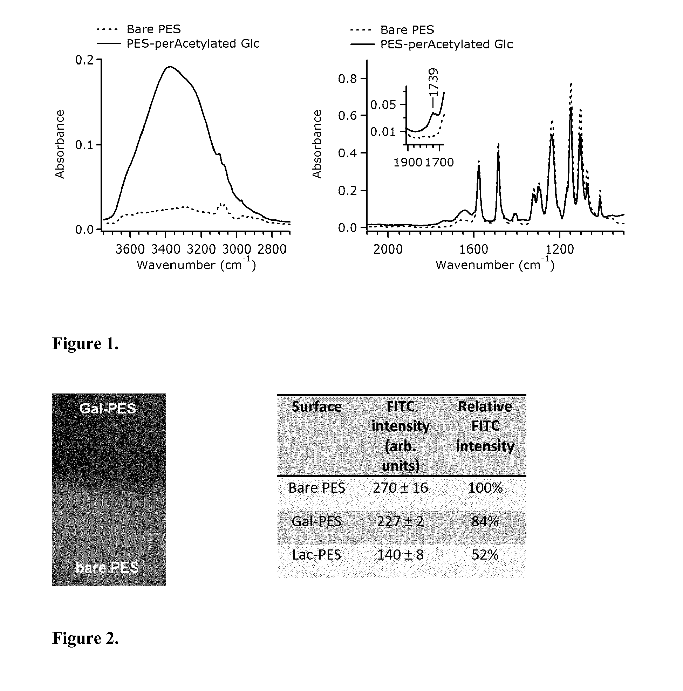

**Figure 1** shows attenuated total reflectance-Fourier transform infrared spectroscopy spectra of PES membranes unmodified (dashed line) and modified with the aryldiazonium cation of compound 4 (continuous line). The inset shows the difference between the two spectra in the carbonyl stretching region, where a C=O stretching peak is visible in the case of membranes modified with per-acylated glucoside groups.
**Figure 2** shows fluorescence images of PES membranes modified with the aryldiazonium cation of galactose compound 1 (Gal-PES) over selected regions, after incubation in Fluorescein isothiocyanate labelled bovine serum albumin (FITC-BSA) solutions (image width = 550 µm). The region that was not functionalised with galactose appears brighter in the image, thus indicating greater BSA adsorption.
**Figure 2** also shows a table with a comparison of average intensity measured at membranes modified with the aryldiazonium cation of galactose compound 1 (Gal-PES) and with the aryldiazonium cation of lactose compound 3 (Lac-PES) compared to that observed on bare (unmodified) PES membranes. Lactose functionalised PES proved significantly more resistant to BSA adsorption than either Galactose functionalised PES or unmodified PES.

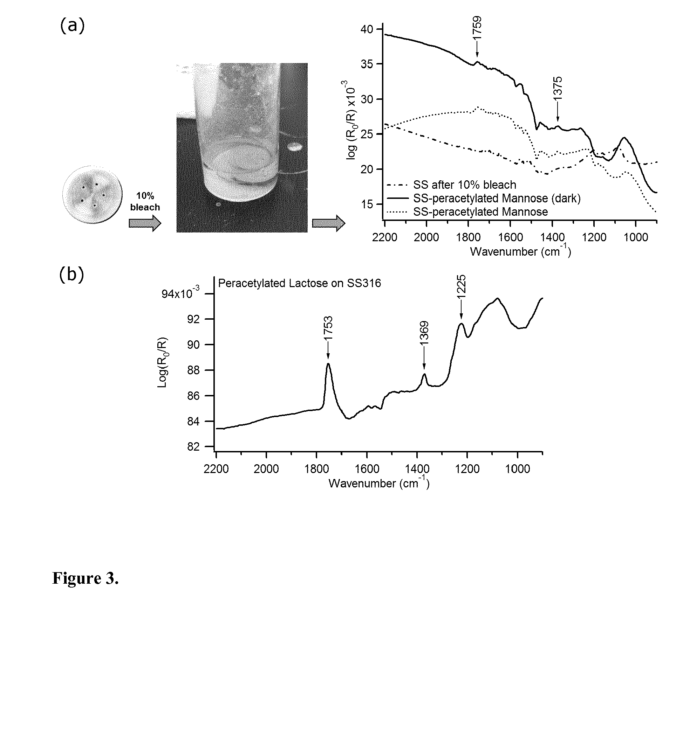

**Figure 3a****:** Modification of stainless steel (SS) restrictor disk, used in beer dispensing taps, with Man-aryldiazonium salt via 10% bleach oxidation followed by immersion in diazonium salt (the aryldiazonium cation of mannopyranose compound 2) solution. Infrared reflection absorption spectroscopy (IRRAS) spectra show a SS disk treated with bleach alone **(dot-dashed** - ratioed against another disk), a SS disk modified with 1.0 mM peracetylated Man-aryldiazonium cation kept in the dark (**continuous** - ratioed against a disk treated in bleach), a SS disk modified with 1.0 mM peracetylated Man-aryldiazonium cation (**dotted** - ratioed against a disk treated in bleach).
**Figure 3b****:** Modification of an SS316 stainless steel plate, after soaking in 10% bleach, with 1.0 mM peracetylated lactose-aryldiazonium cations generated in situ; Infrared reflection absorption spectroscopy **(IRRAS)** spectra obtained using an unmodified SS316 plate as background clearly show the signature of C=O and C-O stretching modes (1753 and 1225 cm⁻¹), and of CH₃ deformation modes (1369 cm⁻¹) characteristic of the acetyl protecting group.
**Figure 4****:** Timeline of immersion experiments using canned beer. Six 15 cm long tubing samples were removed at each time point, 3 of these were uncoated, control samples and 3 of these were Lactose-coated segments.
**Figure 5****:** ATP content data for triplicate samples of Lactose-coated and uncoated segments after 5 and 10 days of immersion in draught beer. Lactose-coated nylon tubing was formed by immersing nylon tubing in a solution of the aryldiazonium cation of compound 3.
**Figure 6****:** ATP content, measured in relative luminescence units (RLU), as a function of immersion time in beer for both control and Lactose-coated nylon tubing segments.
**Figure 7****:** He-ion images of the tubing surface as a function of immersion time in beer for both control and Lactose-coated nylon tubing segments. Deposits increase more rapidly on the uncoated surface than on the coated one.
**Figure 8****:** Test beer dispensing system installed in our laboratory for carrying out 10-day tests. A schematic of the connections is also shown.
**Figure 9****:** ATP content data for triplicate pairs of Lactose-coated and uncoated segments after 10 days of testing in a beer dispensing system (1 pint/day drawn). The amount of ATP accumulated in the tubing decreases after every water rinse, however, on average ATP concentration is lower inside coated tubing.
**Figure 10****:** He-ion microscopy images of lactose modified and uncoated nylon tubing exposed to beer for 10 days (image width = 100 µm); images show negligible bacterial accumulation on lactose modified nylon tubing compared to bare nylon tubing under the same exposure conditions.

### Detailed Description

It should be readily apparent to one of ordinary skill in the art that the examples disclosed herein represent generalised examples only, and that other arrangements and methods capable of reproducing the invention are possible and are embraced by the present invention.

In order to initially develop a biofouling resistant surface, several surfaces were functionalised with different classes of carbohydrate and proof of concept studies were carried out to determine which class of carbohydrate proved most effective.

In order to successfully develop biofouling resistant beverage dispensing equipment, by functionalising the surfaces of equipment/materials currently employed in beverage dispensing equipment, it would be desirable not to have to pre-treat said surfaces to modify the functionality thereon. Notwithstanding the foregoing, if modification of surface functionality is required in advance of tethering carbohydrates thereto, desirably, such modification will not have a deleterious impact on the quality or taste of beverage which comes into contact with said surface.

For example, functionalization of articles having surfaces, by pre-treating the surface to modify the surface functionality, for example, by introducing reactive sulfur groups onto the surface, and then subsequently carrying out a coupling reaction between a functionalised carbohydrate and said surface was not considered a desirable approach for developing biofouling resistant beverage dispensing equipment.

Preferably, surfaces are directly functionalised, without the necessity of pre-treatment.

More, preferably, the carbohydrate is linked to the surface via a linker moiety comprising a carbon atom that forms a covalent bond with an atom on the surface.

It is imperative that the surface thus formed has biofouling resistant properties and that said surface does not comprise potential contaminants which would adversely affect the quality or taste of the beverage which is dispensed through the beverage dispensing equipment. Accordingly, pre-functionalization of surfaces with for example sulfur or the like is not suitable, as such surfaces would inevitably have a deleterious effect on the quality of the beverage dispensed through the equipment and in particular on the taste and fragrance of the beverage.

Cognisant of the above requirements, the linking strategy described below was employed to functionalise a variety of surfaces with varying types of carbohydrates and the biofouling resistant properties of said surfaces was subsequently determined.

The following mono and di-saccharide 4-aminophenyl glycosides were prepared:

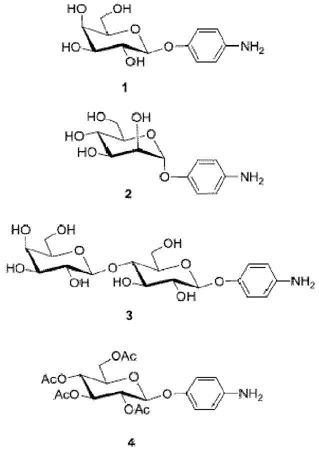

Mono- and di-saccharide 4-aminophenyl glycosides were prepared through the general synthetic pathway outlined in Scheme 1 below:

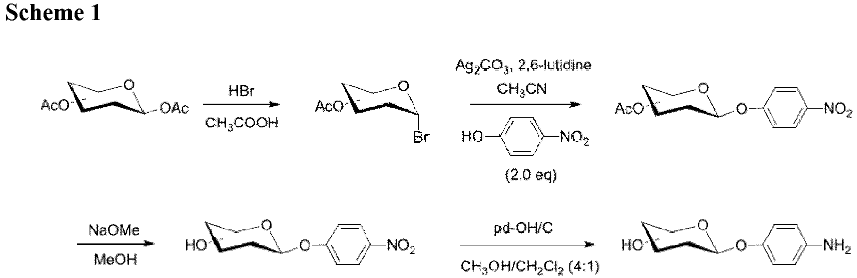

The mono- and di-saccharide 4-aminophenyl glycosides were then used for the modification of PES membranes surfaces *via* immersion into their corresponding aryldiazonium cation solutions as shown in Scheme 2 below:

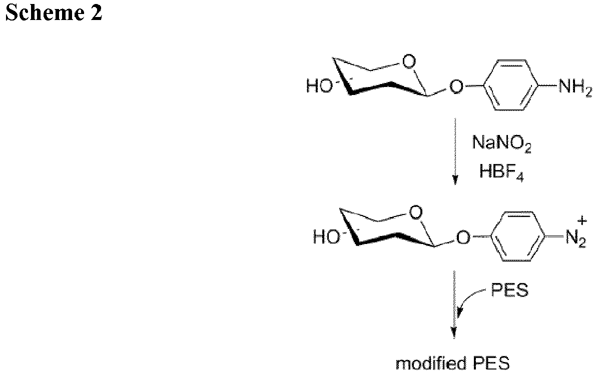

Attenuated Total Internal Reflectance Infrared Spectroscopy (ATR-FTIR) and binding experiments in combination with fluorescence imaging were employed in order to investigate the modification of surfaces.

Figure 1 shows ATR-FTIR spectra of a bare PES membrane (dashed line) and of a PES membrane after modification in a 1.0 mM solution of the aryldiazonium cation of compound **4** (continuous line). The spectrum of the bare PES membrane was found to be in good agreement with PES spectra found in the literature. Two peaks at 3096 cm⁻¹ and 3070 cm⁻¹ are due to aromatic C-H stretching modes, whereas peaks at 1578, 1485 and 1408 cm⁻¹ are attributed to aromatic C=C stretching modes.

Peaks at 1321 and 1297 cm⁻¹ arise from S=O asymmetric stretches in -SO₂-, while the 1146 cm⁻¹ peak is attributed to the symmetric stretching mode. The peak at 1236 cm⁻¹ is characteristic of PES and is usually assigned to the asymmetric stretching of Ar-O-Ar ethers. Broad peaks at 3400 cm⁻¹ and 1640 cm⁻¹ can be attributed to stretching and bending modes of residual water held within the membrane, respectively. A summary of infrared peaks and their assignments is reported in Table I. The spectrum of the PES membrane modified with per-acetylated glucopyranoside **(4)** displays a similar spectral profile to that of bare PES with the exception of two additional peaks at 1739 cm⁻¹ (inset of Figure 1) and 1370 cm⁻¹ characteristic of C=O stretching modes of esters and of -CH₃ bending modes, respectively, which can be attributed to surface-bound acetyl groups.

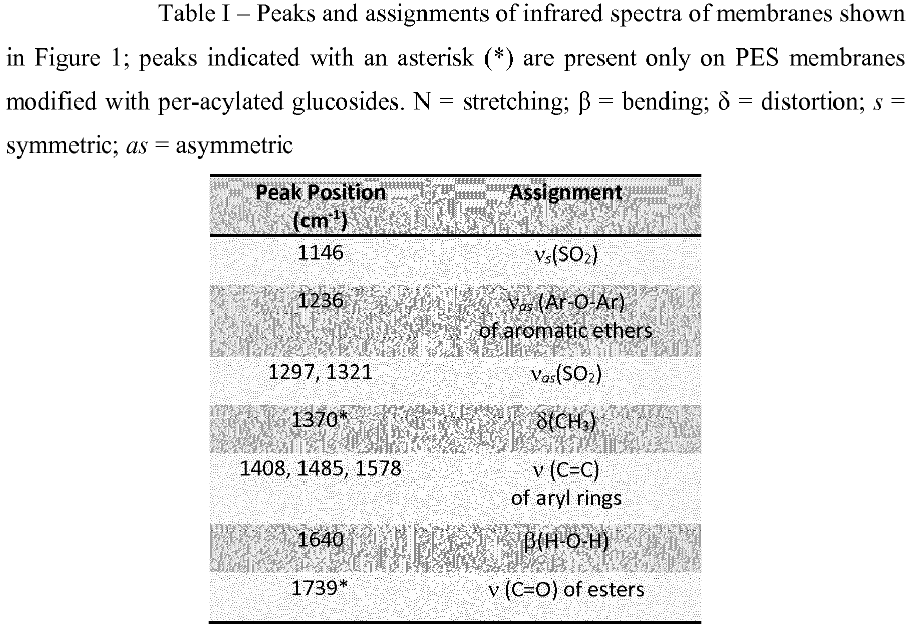

The presence of these peaks indicates that, after immersion of PES membranes in the aryldiazonium cation solution, their surface is modified with per-acetylated glucopyranoside moieties. ATR-FTIR spectra of PES membranes modified with de-acetylated 4-aminophenyl glycosides **1-3** did not reveal significant differences compared to that of a bare PES membrane due to spectral overlap in the mid-infrared between PES and phenyl glycosides.

ATR-FTIR confirmed that aryldiazonium cations can be used for the modification of PES membranes in a single step *via* immersion into solutions of cations prepared *in situ.*

As outlined above, desirably this methodology does not require extensive pre-treatment of surfaces, as direct functionalisation is accomplished.

In order to investigate the potential of carbohydrate functionalised surfaces as biofouling resistant surfaces, we carried out proof of concept studies.

Protein adsorption experiments using Bovine Serum Albumin (BSA) solutions were carried out, to determine:
(i) that the following type of carbohydrate functionalised surfaces are biofouling resistant surfaces - namely: surfaces having a carbohydrate immobilised thereon, wherein the carbohydrate has a linker moiety covalently bound thereto, the linker moiety is disposed between the surface and the carbohydrate, and the linker moiety comprises a carbon atom that forms a covalent bond with an atom on the surface; and
(ii) what class of carbohydrate immobilised on such surfaces proved the most effective biofouling resistant surface.

**Preparation of carbohydrate functionalised surfaces**

**Preparation of the carbohydrate-aryldiazonium derivatives** Carbohydrate-aryldiazonium derivatives were prepared from their corresponding amines according to reported literature procedures: D'Amour, M.; Belanger, D., Stability of Substituted Phenyl Groups Electrochemically Grafted at Carbon Electrode Surface Journal of Physical Chemistry B 2003, 107 (20), 4811-4817; and Hermans, A.; Seipel, A. T.; Miller, C. E.; Wightman, R. M., Carbon-Fiber Microelectrodes Modified with 4-Sulfobenzene Have Increased Sensitivity and Selectivity for Catecholamines. Langmuir 2006, 22 (5), 1964-1969.

In short, the amine derivative of the desired compound (X M) was dissolved in tetrafluoroboric acid (2X M). Following cooling to -5 °C a 1X M solution of sodium nitrite in water was added dropwise over 30 min with stirring. If the tetrafluoroborate salt of the carbohydrate-aryldiazonium derivatives is to be isolated, the precipitate obtained is filtered by suction, washed with an ice cold ether/methanol mixture (4:1) and cold ethanol, and dried under high vacuum.

### Synthesis of carbohydrate-aryldiazonium salt precursors and surface modification.

4-aminophenol-β-D-galactopyranose **(1)** and 4-aminophenol-α-D-mannopyranose precursors (2), shown above were synthesized using established methods. The disaccharide lactose derivative, 4-aminophenol-β-D-lactopyranose **(3)** was synthesised using a similar synthetic approach. Commercially available D-Lactose was per-acetylated on treatment with sodium acetate and acetic anhydride. The product was converted to the glycosyl bromide on treatment with hydrogen bromide in acetic acid. Silver ion promoted glycosylation of the anomeric bromide, furnished the 4-nitrophenol lactose derivative which was subsequently deprotected under basic conditions, and the nitro group reduced to give the desired 4-aminophenol product **3.** Per-acetylated 4-aminophenol-β-D-glucopyranose **(4)** was synthesized using established methods.

### Functionalisation of carbon or metal substrates with disaccharide-aryldiazonium derivatives

### (a) From the isolated tetrafluoroborate salt

A solution of the carbohydrate-diazonium tetrafluoroborate of known concentration, generally in the mM range, is made up in a suitable solvent (water if the compound is water soluble; or, acetonitrile, tetrahydrofuran and alcohols). A clean substrate is immersed in the solution for a noted period of time (typically 5-60 min). Following immersion, the substrate is removed from the reaction vessel, rinsed a number of times in a suitable solvent, sonicated for 30 seconds to remove physisorption products and dried under Argon.

### (b) Through in-situ generation of carbohydrate-diazonium

Carbohydrate-diazonium cations are generated *via in-situ* diazotisation of carbohydrate-arylamine derivatives. The diazoniation procedure is carried out as described above (a) however in this protocol the isolation step is replaced with immediate functionalisation of carbon or metal surfaces. These substrates are immersed in the carbohydrate diazonium cation solution for a period of time (typically 5-60 min), removed and washed in a suitable solvent and finally dried under Argon.

### PES functionalised surface

PES (0.45 µm, 25 mm) membranes were purchased from Sigma Aldrich (Part# Z269247, Mnfr. Pall Corporation). Prior to functionalization they were rinsed twice in deionised water and dried under vacuum and nitrogen flow. They were subsequently immersed for 1 h in the dark at room temperature in a 1.0 mM aqueous solution of the Lac-aryldiazonium cation which was prepared in situ. Samples were then twice washed in deionised water and dried under nitrogen prior to further use.

### Protein binding resistance of carbohydrate functionalised surfaces

Modified and unmodified PES membranes were incubated for 2 h in a 0.2 mg mL⁻¹ solution of FITC-BSA in PBS at pH 7.4; the membranes were then washed with the PBS solution in order to remove unbound protein prior to imaging. Figure 2 shows a fluorescence microscopy image of a PES coated membrane, where only the lower half of the area shown was modified with β-galactose using compound **1** as a precursor. A comparison of the emission intensity levels between the two sample regions clearly shows that the Gal-PES area of the sample displays much lower FITC emission than the unmodified area, thus indicating that modification with Gal layers significantly reduces BSA adsorption from solution.

In order to compare the effectiveness at minimizing BSA adsorption of a mono- and a di-saccharide, we carried out fluorescence imaging of Gal-PES, Lac-PES and bare PES membranes. Modified and unmodified PES membranes were incubated for 2 h in 0.5 mg mL⁻¹ solutions of FITC-BSA in PBS at pH 7.4, rinsed in PBS buffer and then imaged via fluorescence microscopy under identical conditions. Figure 2 also shows a table summarising the FITC emission intensity measured at bare PES, Gal-PES and Lac-PES membranes, after incubation in FITC-BSA. The emission is the highest for bare membranes and is reduced by 16% for Gal-PES surfaces and by 48% for Lac-PES under the experimental conditions.

Thus disaccharide functionalised surfaces proved significantly more effective at minimising protein adsorption than monosaccharide functionalised surfaces.

Our proof of concept study thus determined that carbohydrate functionalised surfaces, linked as described above do have biofouling resistant properties and also said study identified that disaccharides and lactose functionalised surfaces in particular were significantly more resistant to protein adsorption than monosaccharide functionalised surfaces.

### Disaccharide functionalised beverage dispensing equipment

The ability to functionalise beverage dispensing equipment with disaccharides was then investigated.

A general schematic for the synthesis of beverage dispensing equipment having surfaces with disaccharides immobilized thereon is given in Scheme 3 below. 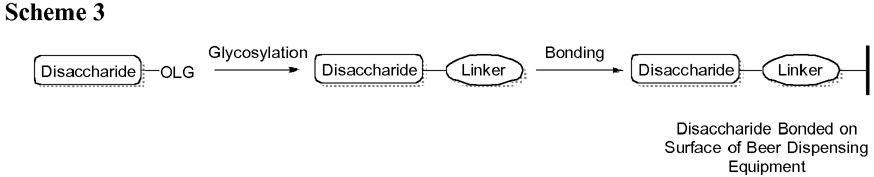 In the present application LG represents a leaving group including halides. Any suitable leaving group can be utilised. In the present application OLG represents a leaving group bonded through an oxygen atom such as tosylate, mesylate, acetate, benzoate, or hydroxide. (It will be appreciated that where the leaving groups in various reaction schemes herein are depicted as OLG, the reaction schemes apply more generically to all suitable leaving groups including those not bonded through an oxygen atom and including for example halides.)

One particularly desirable method for functionalizing the surface of beverage dispensing equipment is to immobilize the disaccharide on said surface via a linker moiety covalently bound to the disaccharide, wherein the linker is disposed between the surface and the disaccharide and the linker moiety comprises a carbon atom that forms a covalent bond with an atom on the surface, wherein the carbon atom of the linker moiety that forms a covalent bond with an atom on the surface is not substituted with =O, =N or =S and wherein the carbon atom in the linker moiety that forms a covalent bond with an atom on the surface is a component of an aromatic or aryl ring. Optionally such immobilization can be achieved by the method comprising:
(i) providing a disaccharide having a linker moiety covalently bound thereto, wherein the linker moiety comprises a carbon atom bonded to a diazonium cation, and wherein the carbon atom in the linker moiety that is bonded to the diazonium cation is a component of an aromatic or aryl ring such that the diazonium cation is an aryl diazonium cation; and
(ii) reacting the diazonium cation with the surface, such that reduction of the diazonium cation results in the carbon atom of the linker moiety forming a covalent bond with an atom on the surface.

As used herein, a diazonium cation bonded to a carbon atom refers to a compound of the formula: 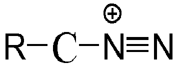 wherein R represents the remainder of the linker moiety. The diazonium cation may have an associated anion. Suitable anions include organic anions, and inorganic anions. For example, halide anions or BF₄⁻.

The carbon atom in the linker moiety may be a component of an aromatic or aryl ring and the diazonium cation may be an aryl diazonium cation. As will be understood by the skilled person when reading that the carbon atom in the linker moiety may be a component of an aryl ring, it will be understood that the carbon atom forms part of the aryl ring itself. For example, suitable aryl diazonium cations include phenyl diazonium cations of the formula: 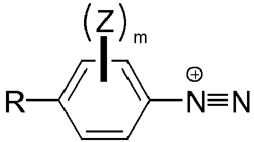 wherein R represents the remainder of the linker moiety
m can be 0 to 4; and
each Z can be independently selected from hydroxy, Cl, Br, I, F,
cyano, C₁-C₅ alkoxy, and C₁-C₅ thioalkoxy.

As outlined above, it is imperative that the linking strategy employed, is facile, can be achieved without requiring chemical pre-treatment of surfaces of beverage dispensing equipment, that the disaccharide remains bound to the surface via a robust linkage i.e. a covalent bond, and that the linker and/or linking strategy does not compromise the quality or taste of the beverage which will be dispensed through the beverage dispensing equipment.

A general schematic for the synthesis of the amino precursors to diazonium substituted disaccharides is given in Scheme 4 below, wherein 4-nitro benzyl alcohol is employed as a representative linker.

### Scheme 4

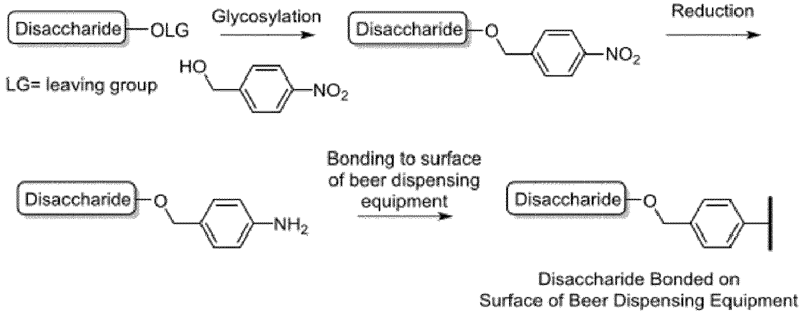

In particular, the anomeric position on the disaccharide is substituted with a leaving group, e.g. bromide. The leaving group is eliminated and replaced by an aromatic nitro compound, e.g. wherein a nitrophenyl ring is substituted with an aliphatic chain terminating with oxygen. The nitro functional group is subsequently reduced to the corresponding amine, whereupon it is subjected to a diazotisation procedure.

The resulting carbohydrate diazonium material can be isolated (and stored for subsequent use at a later stage) or carried on to the next stage of the reaction process, *i.e.* reaction with the surface of the beverage dispensing equipment such that the carbon atom of the linker bonded to the diazonium cation now becomes bonded to the surface.

The immobilization strategy outlined in Scheme 4 above was employed to functionalize beverage dispensing surfaces with disaccharides and surfaces suitable for use in beverage dispensing equipment with disaccharides.

### Example 1 - synthesis of D-lactose bound to aromatic linker

Commercially available D-Lactose was acetylated using sodium acetate and acetic anhydride. The product was converted to the lactosyl bromide using Hydrogen bromide in acetic acid. Silver ion mediated glycosylation of the bromide produced the 4- Nitrophenyl lactose derivative which was deprotected and reduced to give the final product (Scheme 5).

### Scheme 5

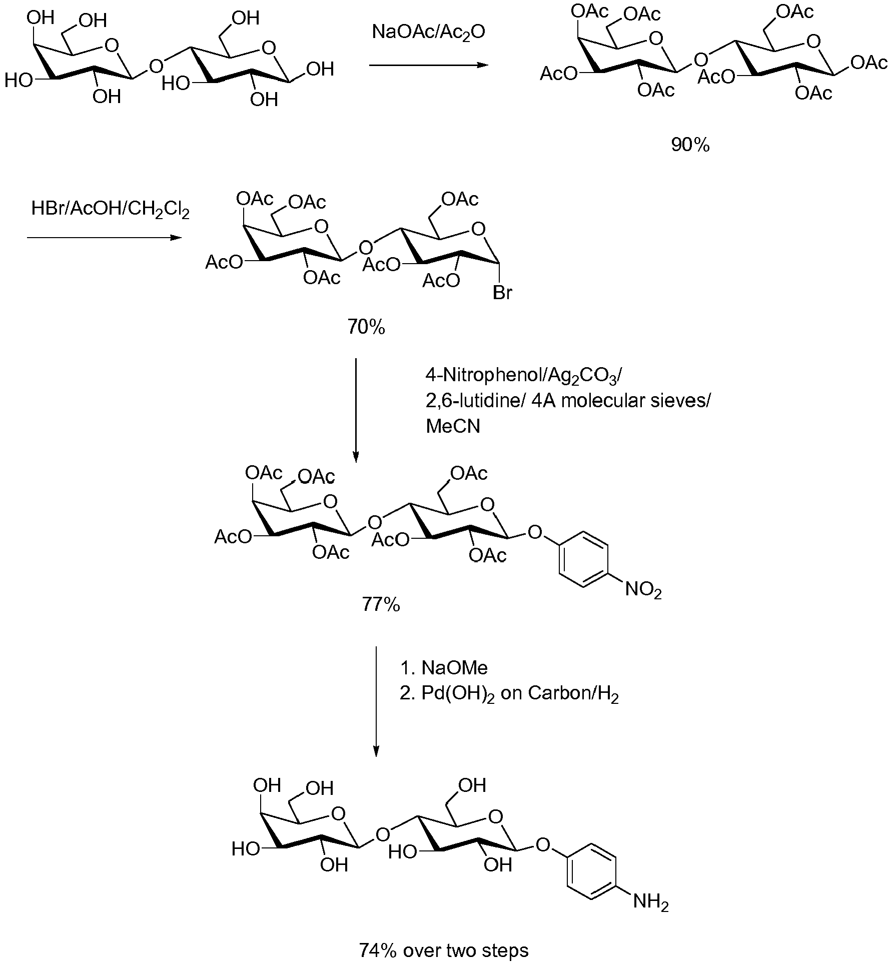

### 4-Nitrophenyl-2,3,4,6-tetra-O-acetyl-β-D-galactopyranosyl-(1→4)-2,3,6-tri-O-acetyl-β-D-glucopyranoside (3a)

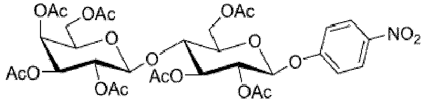

1-Bromo-2,3,4,6-tetra-*O*-acetyl-β-D-galactopyranosyl-(1→4)-2,3,6-tri-*O*-acetyl- -D-Glucopyranoside (20 g, 28.65 mmol), 4-Nitrophenol (8 g, 57.3 mmol), Silver carbonate (15.8 g, 57.3 mmol) and 4A molecular sieves (5 g) were dried under high vacuum for 1 h. To this was added acetonitrile, (100 ml, dried over 3A molecular sieves, sieves included) followed by 2,6-Lutidine (7 ml, 57.3 mmol) and the mixture was stirred at RT for 16 h in the dark. The mixture was filtered through a plug of (diatomaceous material (CAS no. 68855-54-9) Celite™ and the filter cake washed with MeCN (2 x 100 ml). The combined filtrate was concentrated to remove MeCN and the oily residue dissolved in dichloromethane, (100 ml). This was washed with saturated NaHCO₃ (3 x 100 ml), brine, water, 1 M HCl and finally water, and dried using MgSO₄. The solution was concentrated to approximately 20 ml and then added dropwise to petroleum ether (300 ml) at -20 °C followed by cooling at -10 °C for 12 h. The solid was filtered and dried. Cream solid (16 g, 74%). [α]_{D20} (deg cm₃ g₋₁ dm₋₁) = -38.0° (c = 2.5 x 10₋₃ g cm₋₁ in CDCl₃); ₁H-NMR (600 MHz, CDCl₃), δ 8.17 (2H, d, *J=* 9.0 Hz, Ar-H2, H3), 7.02 (2H, d, *J=* 9.0 Hz, Ar-H1, H4), 5.37 (1H, d, *J*_{*4*,*3*} = 3.4 Hz, H4-Gal), 5.31 (1H, m, H3-Glc), 5.23-5.19 (2H, m, H2-Glc, H1-Glc), 5.13 (1H, dd, *J_{2,1} =* 8.0 Hz, *J_{2,3} =* 10.4 Hz, H2-Gal), 4.98 (1H, dd, *J_{3,4} =* 3.4 Hz, *J_{3,2} =* 10.5 Hz, H3-Gal), 4.53 (1H, d, *J_{1,2}* = 8.0 Hz, H1-Gal), 4.51 (1H, m, H6a-Glc), 4.18- 4.12 (2H, m, H6b-Glc, H6a-Gal), 4.09 (1H, dd, *J_{6b,5}* = 7.5 Hz, *J_{6b,6a}* = 11.2 Hz, H6b-Gal), 3.94-3.90 (2H, m, H5-Gal, H5-Gal), 3.87 (1H, m, H4-Glc), 2.16, 2.09, 2.07, 2.07, 2.06, 2.06, 1.97 (21H, 7 x CH₃, OAc); ₁₃C-NMR (125 MHz, D₂O); δ 170.3, 170.0, 170.0, 169.9, 169.6, 169.4, 169.0 (7 x C=O), 161.1, 143.2 (2 x qC), 125.7, 116.5 (2 x Ar CH), 101.1 (C-1, Gal), 97.7 (C-1, Glc), 76.0 (C-5, Glc), 73.1 (C-4, Glc), 72.5 (C-3, Glc), 71.2 (C-2, Glc), 70.8 (C-3, Gal), 70.7 (C-5, Gal), 69.0 (C-2, Gal), 66.6 (C-4, Glc), 61.8 (C-6, Glc), 60.8 (C-6, Gal), 20.7 , 20.6, 20.5, 20.5, 20.5, 20.5, 20.3 (7 x CH₃ OAc): HRMS (ESI, m/z): [M+Na]+ calculated for C₃₂H₃₉NO₂₀Na = 780.1957. Found 780.1983. IR (Thin film) ν ₘₐₓ (L) 3100 (NH₂), 1736 (C=O), 1214 (C-O), 1034 (C-O) cm⁻¹.

### 4-Nitrophenyl-β-D-galactopyranosyl-(1→4)-2,3,6-tri-O-acetyl-β-D-glucopyranose (3b)

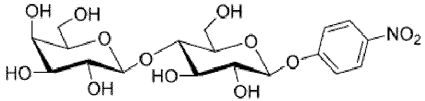

Compound (3a) (11 g, 14.53 mmol) was slurried in methanol (MeOH, 150 ml) and 1 M sodium methoxide solution in MeOH was added dropwise until the mixture was at pH 10. The solid slowly dissolved and the mixture was stirred at room temperature for 16 h. The reaction mixture was adjusted to pH 7 with dropwise addition of 6M HCl. The solid product was filtered, washed with MeOH and dried. White solid (6.5 g, 97 %). [α]_{D20} (deg cm₃ g₋₁ dm₋₁) = -70.0° (c = 2.0 x 10₋₃ g cm₋₁ in MeOH/H₂O 1:1); ₁H-NMR (600 MHz, d₆-DMSO), δ 8.22 (2H, d, *J=* 9.0 Hz, Ar-H2, H3), 7.25 (2H, d, *J=* 9.0 Hz, Ar-H1, H4), 5.60 (1H, d, *J =* 5.2 Hz, OH) 5.20 (1H, *d, J =* 7.8 Hz, H1-Glc), 4.84 (1H, s, OH), 4.80 (1H, *d, J =* 5.0 Hz, OH) 4.67 (1H, d, *J* = 5.0 Hz, OH), 4.63 (1H, *d, J =* 5.4 Hz, OH), 4.53 (1H, *d, J =* 4.4 Hz, OH), 4.26 (1H, d, *J* = 7.6 Hz, H1-Gal), 3.76 (1H, m, H6ₐ, H6-Glc), 3.65 (1H, m, H6_{b}, H6-Glc), 3.64 (1H, m, H5-Glc), 3.63 (1H, m, H4-Gal), 3.54 (2H, m, H6ₐ, H6_{b}, H6-Gal), 3.50 (1H, m, H3- Glc), 3.45 (1H, m, H5-Gal), 3.44 (1H, m, H4-Glc), 3.34 (1H, m, H2-Gal), 3.35 (1H, m, H2- Glc), 3.32 (1H, m, H3-Gal); 13C-NMR (125 MHz, d₆DMSO); 162.9 (Ar-C1), 141.9 (q-C, Ar-C4), 125.7 (q-C, Ar-C3, C5), 116.5 (Ar-C2, C6), 103.8 (C1-Gal), 99.3 (C1-Glc), 79.9 (C4-Glc), 75.5 (C5-Gal), 75.1 (C5-Glc), 74.6 (C3-Glc), 73.2 (C3-Gal), 72.8 (C2-Glc), 70.5 (C2-Gal), 68.1 (C4-Gal), 60.4 (C6-Gal), 59.9 (C6-Glc); HRMS (ESI, m/z): [M+Na]+ calculated for C₁₈H₂₅NO₁₃Na = 486.1218. Found 486.1485. IR (Thin film) ν ₘₐₓ (L) 3311 (OH), 1597, 1214 (C-O), 1034 (C-O) cm⁻¹.

### 4-Aminophenyl-β-D-galactopyranosyl-(1→4)-2,3,6-tri-O-acetyl-β-D-glucopyranose) (3c)

Compound **(3b)** (1.0 g, 2.16 mmol) was dissolved in a 1:1 mixture of EtOH/H₂O (20 ml) and the solution was degassed with N₂ for 15 min. Pd(OH)₂ on carbon (50 mg) as added and the mixture was further degassed for 10 min. The flask was evacuated and subsequently saturated with H₂ over a period of 2 h after which time TLC indicated complete consumption of starting material. The mixture was filtered through a 0.4 micron filter and the solvent removed to give a solid product. Cream solid (880 mg, 94%). [α]_{D20} (deg cm³ g₋₁ dm₋₁) = - 46.2° (c = 2.1 x 10₋₃ g cm₋₁ in MeOH:H₂O); ₁H-NMR (600 MHz, d₆-DMSO), δ ₁H-NMR (600 MHz, d₆-DMSO), δ 6.77 (2H, d, *J* = 8.3 Hz, Ar-H2, H3), 6.50 (2H, d, *J* = 8.3 Hz, Ar- H1, H4), 5.34 (1H, d, *J=* 5.3 Hz, OH), 5.10 (1H, d, *J=* 4.2 Hz, OH), 4.78 (1H, d, *J* = 4.4 Hz, OH), 4.74 (1H, s, OH), 4.68 (1H, s, OH), 4.67 (1H, d, *J =* 8.0 Hz, H1-Gln), 4.58 (1H, t, *J =* 6.0 Hz, OH), 4.52 (1H, d, *J =* 4.4 Hz, OH), 4.24 (1H, d, *J =* 7.7 Hz, H1-Gal), 3.55-3.52 (2H, m, H6ₐ, H6_{b}-Glc), 3.63 (1H, m, H5-Glc), 3.63 (1H, m, H4-Gal), 3.76-3.64 (2H, m, H6ₐ, H6_{b}, Gal), 3.41 (1H, m, H3-Glc), 3.38 (1H, m, H5-Gal), 3.48 (1H, m, H4-Gln), 3.34 (1H, m, H2- Gal), 3.23 (1H, m, H2-Glc), 3.33 (1H, m, H3-Gal); ₁₃C-NMR (125 MHz, d₆DMSO); 148.0 (q-C, Ar-C1), 143.5 (q-C, Ar-C4), 117.8 (Ar-C3, C5), 114.6 (ArC2, C6), 104.0 (C1-Gal), 102.0 (C1-Glc), 80.4 (C5-Gal), 75.8 (C4-Glc), 65.2 (C5-Glc), 74.9 (C3-Glc), 73.3 (C3-Gal), 73.0 (C2-Glc), 70.5 (C2-Gal), 68.1 (C4-Gal), 60.3 (C6-Gal), 60.4 (C6-Glc); HRMS (ESI, m/z): [M+Na]+ calculated for C₁₈H₂₇NO₁₁Na = 456.1476. Found 456.1455. IR (Thin film) ν ₘₐₓ (L) 3294 (OH), 1226 (C-O), 1090 (C-O) cm⁻¹.

### Disaccharide functionalised beer dispensing equipment

Surfaces suitable for use in beer dispensing equipment were functionalised employing the functionalization strategy outlined above.

### Polyamide functionalised surface

Nylon tubing was obtained in the form of both new tubing (Mnfr. Valpar) or tubing removed from trade i.e. a commercial pub (nylon-11, ID 0.7-1.2 cm). Unless stated, all tubing for fouling experiments originated from trade. Prior to pre-treatment, tubing was either rinsed with water or soaked in caustic cleaning solution used for commercial beer line cleaning (consisting of caustic bleach and surfactants) for 20 min at room temperature and subsequently rinsed with water. Oxidative pre-treatment consisted of filling the tubing arranged in a coil with 37% formaldehyde: 50% hypophosphorous acid (HCHO:H₃PO₂) in 100:1 v/v ratio at 30 °C overnight in a water bath. Prior to functionalization, the tubing was rinsed in deionised water and dried under nitrogen flow; then, it was filled with a 0.1 mM aqueous solution of Lac-aryldiazonium cations and left at room temperature in the dark for 1 h. Tubing was subsequently washed with water and installed in a beer dispensing system.

### Brass functionalised surface

Brass fittings were purchased from Radionics (Part# 369-1586). Prior to functionalization they were washed with soap and water, then sonicated in methanol, etched in a 0.1 M solution of HNO₃ for 5 min, and finally rinsed in water. They were subsequently immersed for 1 h in the dark at room temperature in a 0.5 mM aqueous solution of the aryldiazonium cation which prepared in situ. Samples were then washed in deionised water and dried under nitrogen prior to further use and assembly.

### Stainless steel

Stainless steel is commonly used in beverage dispensing equipment-such as in beer faucets (taps), shanks, and restrictor disks.

**Figure 3a** demonstrates how stainless steel (SS) restrictor disk, used in beer dispensing taps, were modified with carbohydrates employing the same linking strategy as described above. The stainless steel restrictor disks were soaked in 10% bleach and subsequently immersed in Man-aryldiazonium salt solution. IRRAS spectra show a SS disk treated with bleach alone (dashed line - ratioed against another disk), a SS disk modified with 1.0 mM peracetylated Man-aryldiazonium cation kept in the dark (solid line - ratioed against a disk treated in bleach), a SS disk modified with 1.0 mM peracetylated Man-aryldiazonium cation (dotted line - ratioed against a disk treated in bleach). These results indicate that functionalization happens whether the aryldiazonium salt solution is exposed to light or not. Advantageously, the fact that functionalization happens in the same way under both dark and illuminated conditions means it can be used to functionalise piping and other surfaces that are kept in the dark (e.g. cooling coils, valves, and pipes in insulated beer pipelines).

The stainless steel plate was also functionalised with carbohydrates. The stainless steel plate was soaked with 10% bleach, and treated with 1.0 mM peracetylated Lactose-aryldiazonium cations generated in situ; Infrared reflection absorption spectroscopy (IRRAS) spectra obtained using an unmodified SS316 plate as background clearly show the signature of C=O and C-O stretching modes (1753 and 1225 cm⁻¹), and of CH₃ deformation modes (1369 cm⁻¹) characteristic of the acetyl protecting group.

### Results

The performance of articles having disaccharide coatings in antifouling applications in beer dispensing was assessed following two different approaches. First, immersion tests under static conditions; second, tests under flow using a test beer dispensing system.

Immersion tests were carried out under aerobic conditions, by using thirty 15 cm long segments of Valpar® tubing taken from a single beer line removed from trade, pre-cleaning them with cleaning-in-place (CIP) solution (e.g. bleach, base, oxidants, surfactants and combinations thereof) and modifying them with Lac-aryldiazonium as described above. They were then immersed into commercially available canned draught beer inside culture bottles. Segments were kept at room temperature and removed at time intervals to be analysed for:
- **ATP content,** by using Aquasnap Total test kits and the EnSURE luminometer (Hygiena). Tubes were rinsed once with deionised water, then filled with 15 mL of water and the ATP content of the water was analysed with the kit. Three tube segments were used to generate each time point.
- **Bacterial accumulation at the tube surface**, by using He-Ion microscopy. Regions of both control and Lactose-coated tubing were cut open and imaged using He-Ion microscopy in order to qualitatively assess the amount of bacteria remaining on the surface.

### Static tests

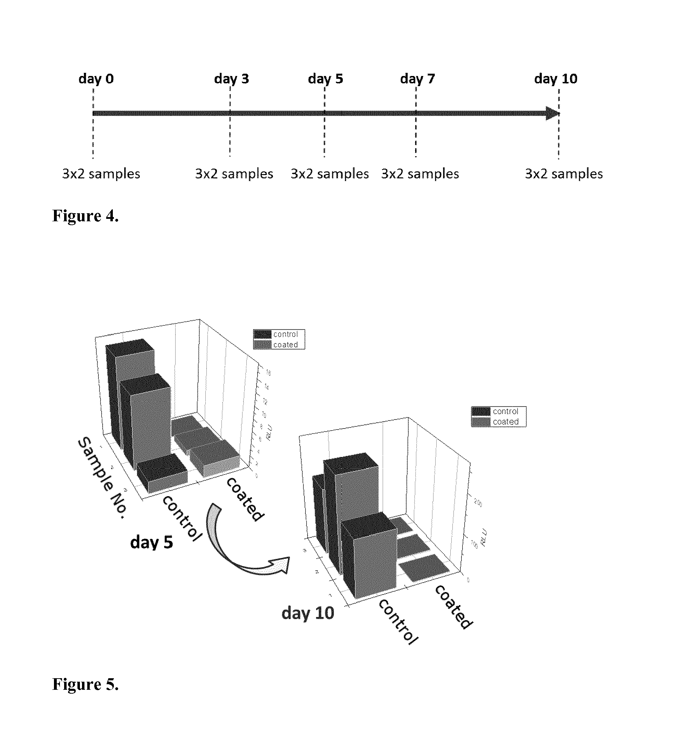

**Figure 4** shows the timeline of these experiments: six 15 cm long tubing samples were removed at each time point, 3 of these were uncoated, control samples, and 3 of these were Lactose-coated segments.

**Figure 5** shows an example of ATP content data for triplicate samples of Lactose-coated and uncoated segments after 5 and 10 days of immersion in draught beer. The data shows that the ATP content in solution is much lower in the case of Lactose-coated tubing than of bare nylon tubing.

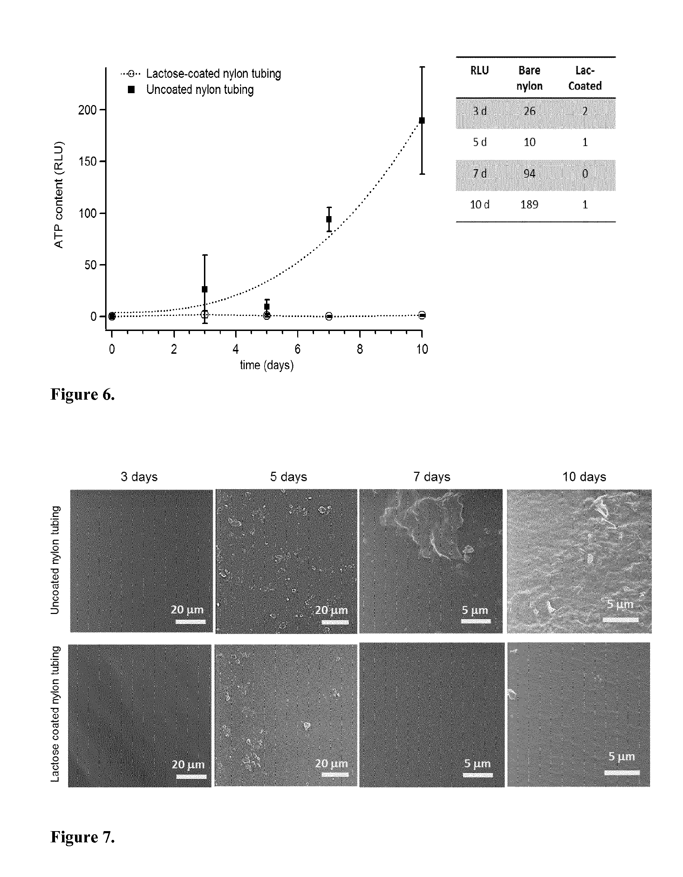

**Figure 6** shows the time evolution of the ATP content measured for nylon tubing: these results show that contamination inside the tube segments builds up much more slowly in Lactose-modified tubing than in bare tubing. This trend was mirrored by microscopy analysis using He-ion microscopy - see Figure 7.

**Figure 7** shows the progression of deposits on the internal surface of the tubing as a function of immersion time in beer. The amount of deposits builds more rapidly on uncoated tubing than on Lactose-coated tubing, thus indicating that the disaccharide coating inhibits the formation of a biofilm.

### Flow tests

Tests under flow were carried out in a beer dispensing system installed in our laboratory. The system consisted of one keg shared among three pairs of taps; each pair was connected to a cooler and two nylon segments, approximately 1 m long, were connected to each tap: one control bare nylon segment and one Lactose-modified nylon segment.

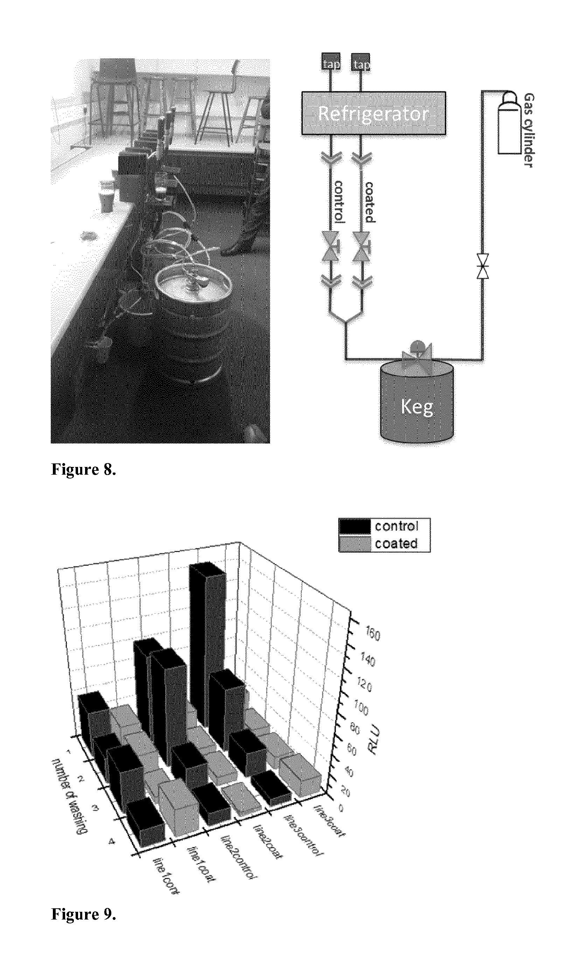

**Figure 8** shows the installation in our laboratory; this is a considered a "worst-case scenario" setup for beer dispensing because of the lack of cooling over the length of the tubing, the necessity to frequently disconnect the keg from the tap head and the consequent exposure to air of the inside surface of the tubing. A pint of beer was drawn every day from each tap over 10 working days; the lines remained empty after each beer dispensing due to the shared keg arrangements.

After a first 10-working day test we concluded that important requirements in order to obtain consistent results are: both control and coated tubing segments must be cleaned at time zero using the CIP solution (20 min soak, followed by rinsing in deionised water), and both segments must originate from the same line from trade in order to be comparable. After CIP cleaning and Lactose-modification of the test nylon segments, these were installed in the system and left at room temperature for 10 working days, while drawing pints daily.

After 10 days the tubing was disconnected from the system and tested in order to determine:
- **ATP content,** by using Aquasnap Total test kits and the EnSURE luminometer (Hygiena). Tubes were rinsed once with deionised water and this water was discarded in order to remove any excess beer. Tubing was then filled with water and the ATP content of the water was analysed with the kit; this process was repeated 3 times.
- **Assessment of bacterial accumulation at the tube surface,** using He-Ion microscopy. Regions of both control and Lactose-coated tubing were cut open and imaged using He-Ion microscopy in order to qualitatively assess the amount of bacteria remaining on the surface.

**Figure 9** shows the result of ATP content measurements obtained after each one of the consecutive four water rinses for both the control, bare nylon, tubing and the Lactose coated nylon tubing. The results indicate that the contamination inside the tubing, as indicated by the ATP concentration, is smaller for lactose coated segments than for uncoated segments. ***Tests showed an average 65% reduction in ATP for Lactose coated lines vs. uncoated lines.***

This result was also consistent with results from He-ion microscopy that show a lower amount of deposits, including bacteria, on coated nylon compared to bare nylon surfaces.

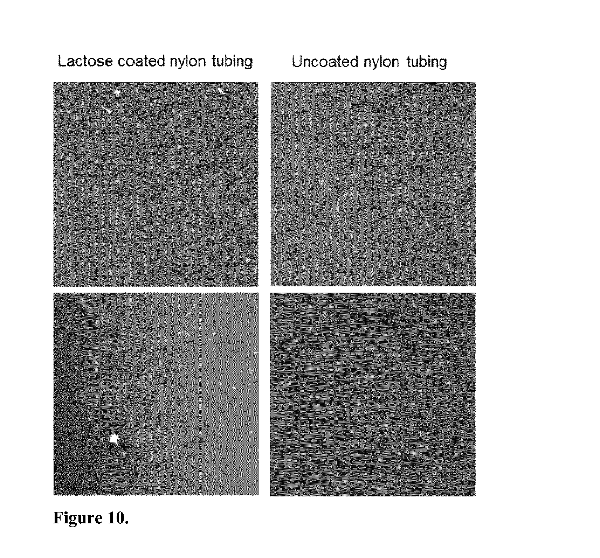

**Figure 10** shows two typical regions of control and test tubing (lactose functionalised nylon) that clearly illustrate the observed difference. Regions closer to the fittings used to connect the tubing segments were found to display greater amounts of deposits.

Overall, disaccharide coated surfaces, and in particular lactose functionalised surfaces proved effective biofouling resistant surfaces.

Beer dispensing equipment having a disaccharide immobilised thereon demonstrated significant resistance to biofouling versus non-functionalised beer dispensing equipment. Lactose functionalised beer dispensing equipment showed an average 65% reduction in ATP content than uncoated beer dispensing equipment.

Advantageously, increasing the biofouling resistance of beer dispensing equipment reduces the frequency at which such equipment requires cleaning. Such improved efficiencies have associated cost benefits.

Significantly, functionalization of beer dispensing equipment with disaccharides as outlined herein, does not negatively impact the quality or taste of the beer which is dispensed through said equipment.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. Use of:
an article, in beverage dispensing equipment;
said article having a surface;
said surface having a disaccharide immobilised thereon,
the disaccharide having a linker moiety covalently bound thereto, the linker moiety disposed between the surface and the disaccharide, and
the linker moiety comprising a carbon atom that forms a covalent bond with an atom on the surface.

2. Use of an article according to claim 1, in beverage dispensing lines and/or beverage dispensing taps.

3. Use of an article according to claim 1, wherein the surface is made substantially from a stainless steel material, a polymethylmethacrylate, a polyamide material, a polyethylene material, a polyacetal material, an acrylonitrile material, and/or a polyvinyl material, optionally wherein the surface is made substantially from nylon.

4. Use of an article according to any preceding claim, wherein the disaccharide is selected from the group consisting of lactose, cellobiose, lactulose, trehalose, laminaribiose and xylobiose.

5. Use of an article according to any preceding claim, wherein the linker moiety comprises a carbon atom that forms a covalent bond with an atom on the surface, wherein the carbon atom of the linker moiety that forms a covalent bond with an atom on the surface is not substituted with a =O, =N or a =S moiety,
wherein the carbon atom in the linker moiety that forms a covalent bond with an atom on the surface is a component of an aromatic or aryl ring,
optionally wherein the linker moiety is: a C₅-C₁₀ aryl group for example a C₆ aryl group.

6. Use of an article according to any preceding claim wherein the beverage dispensing equipment is beer dispensing equipment; or cider dispensing equipment; or soft drink dispensing equipment; or dairy dispensing equipment.

7. A surface having a disaccharide immobilised thereon,
the disaccharide having a linker moiety covalently bound thereto, the linker moiety disposed between the surface and the disaccharide, and the linker moiety comprising a carbon atom that forms a covalent bond with an atom on the surface, wherein the carbon atom of the linker moiety that forms a covalent bond with an atom on the surface is not substituted with a =O, =N or a =S moiety,
wherein the carbon atom in the linker moiety that forms a covalent bond with an atom on the surface is a component of an aromatic or aryl ring,
wherein the disaccharide is selected from the group consisting of:
lactose, cellobiose, lactulose, trehalose, laminaribiose and xylobiose; and
the linker moiety is a C₅-C₁₀ aryl group.

8. A surface according to claim 7, wherein the surface is made substantially from a stainless steel material, a polymethylmethacrylate, a polyamide material, a polyethylene material, a polyacrylic material, an acrylonitrile material, and/or a polyvinyl material.

9. A surface according to claim 8, wherein the surface is made substantially from a polyamide material, for example nylon.

10. A surface according to any one of claims 7 to 9, wherein the disaccharide is lactose.

11. Use of a surface according to any one of claims 7 to 10 in beer dispensing equipment.

12. Beverage dispensing equipment having a surface; said surface having a disaccharide immobilised thereon; wherein the disaccharide has a linker moiety covalently bound thereto; the linker moiety is disposed between the surface and the disaccharide, and the linker moiety comprises a carbon atom that forms a covalent bond with an atom on the surface.

13. Beverage dispensing equipment according to claim 12, wherein the surface is made substantially from a stainless steel material, a polymethylmethacrylate, a polyamide material, a polyethylene material, a polyacetal material, an acrylonitrile material, and/or a polyvinyl material; and/ or
wherein the surface is made substantially from nylon, for example, nylon-4,6, nylon-6,6, nylon-6, nylon-11, or nylon-12; and/or
wherein the disaccharide is selected from the group consisting of lactose, cellobiose, lactulose, trehalose, laminaribiose and xylobiose, for example lactose, and/or
wherein the linker is a C₅-C₁₀ aryl group; and/or
wherein the disaccharide is lactose and the linker is a C₅-C₁₀ aryl group; and/or wherein the surface is made substantially from a stainless steel material, a polyamide material, a polyethylene material, a polyacetal material, and/or a polyvinyl material.

14. Beer dispensing equipment having a surface; said surface having a disaccharide immobilised thereon; wherein the disaccharide has a linker moiety covalently bound thereto; the linker moiety is disposed between the surface and the disaccharide, and the linker moiety comprises a carbon atom that forms a covalent bond with an atom on the surface.

15. Beer dispensing equipment according to claim 14, wherein the surface is made substantially from a stainless steel material, a polymethylmethacrylate, a polyamide material, a polyethylene material, a polyacetal material, an acrylonitrile material, and/or a polyvinyl material; and/or
wherein the surface is made substantially from nylon, for example, nylon-4,6, nylon-6,6, nylon-6, nylon-11, or nylon-12; and/or
wherein the disaccharide is selected from the group consisting of lactose, cellobiose, lactulose, trehalose, laminaribiose and xylobiose, for example lactose; and/or
wherein the linker is a C₅-C₁₀ aryl group; and/or
wherein the disaccharide is lactose and the linker is a C₅-C₁₀ aryl group; and/or
wherein the surface is made substantially from a stainless steel material, a polyamide material, a polyethylene material, a polyacetal material, and/or a polyvinyl material.
